# EUROPEAN PATENT APPLICATION

(11) **EP 3 804 751 A2**
(43) Date of publication of application: **14.04.2021**
(21) Application number: 20829299.5
(22) Date of filing: 19.08.2020
(51) Int. Cl.: A61K 39/215, A61P 31/14, C12N 15/861

(54) **ADENOVIRUS CARRIER VACCINE USED FOR PREVENTING SARS-COV-2 INFECTION**

(30) Priority: 23.02.2020 CN 202010110070; 05.03.2020 CN 202010145657
(71) Applicant: Guangzhou N Biomed Ltd., Guangzhou, Guangdong 510663 (CN)
(72) Inventor: CHEN, Ling, Guangzhou, Guangdong 510663 (CN); GUAN, Suhua, Guangzhou, Guangdong 510663 (CN); YANG, Chenchen, Guangzhou, Guangdong 510663 (CN); LIU, Xiaolin, Guangzhou, Guangdong 510663 (CN)
(74) Representative: Gassner, Birgitta
(86) International application number: PCT/CN2020/110085
(87) International publication number: WO 2021/000968

(57) **Abstract**

The present disclosure discloses an adenovirus vectored vaccine for preventing SARS-CoV-2 infection, which comprises a nucleotide sequence as shown by SEQ ID NO: 1. According to some embodiments of the present disclosure, the vaccine comprises an S protein-coding nucleotide sequence which is easily expressed in human cells, and can produce more S proteins. The vaccine of the present disclosure is expected to be used as a recombinant viral vaccine for preventing SARS-CoV-2 infection. Some embodiments of the present disclosure have better safety.

SEQUENCE LISTING

<110>  GUANGZHOU N BIOMED LTD.

<120>  ADENOVIRUS-VECTORED VACCINE FOR PREVENTING SARS-COV-2 INFECTION

<130>   P20GZ1NW00084US

<150>  CN202010110070.8
<151>  2020-02-23

<150>  CN202010145657.2
<151>  2020-03-05

<160>  10

<170>  PatentIn version 3.5

<210>  1
<211>  3822
<212>  DNA
<213>  Artificial Sequence

<220>
<223>  CDS

<400>  1
atgttcgtgt ttctggtgct gctgcctctg gtgagctccc agtgcgtgaa cctgaccaca      60

aggacccagc tgccacctgc ctataccaat agcttcacac ggggcgtgta ctatcccgac     120

aaggtgttta gatctagcgt gctgcactcc acccaggatc tgtttctgcc tttcttttct     180

aacgtgacat ggttccacgc catccacgtg tccggcacca atggcacaaa gcggttcgac     240

aatccagtgc tgccctttaa cgatggcgtg tacttcgcct ccaccgagaa gtctaacatc     300

atcagaggct ggatctttgg caccacactg gacagcaaga cccagtccct gctgatcgtg     360

aacaatgcca caaacgtggt catcaaggtg tgcgagttcc agttttgtaa tgatcccttc     420

ctgggcgtgt actatcacaa gaacaataag tcttggatgg agagcgagtt tagggtgtat     480

tcctctgcca acaattgcac ctttgagtac gtgagccagc ctttcctgat ggacctggag     540

ggcaagcagg gcaatttcaa gaacctgagg gagttcgtgt ttaagaatat cgatggctac     600

ttcaagatct actccaagca cacaccaatc aacctggtgc gcgacctgcc acagggcttc     660

tctgccctgg agccactggt ggatctgccc atcggcatca acatcacccg gtttcagaca     720

ctgctggccc tgcacagaag ctacctgacc ccaggcgaca gctcctctgg atggacagca     780

ggagctgccg cctactatgt gggctatctg cagccccgca ccttcctgct gaagtacaac     840

gagaatggca ccatcacaga cgcagtggat tgcgccctgg accccctgtc tgagaccaag     900

tgtacactga agagctttac agtggagaag ggcatctacc agaccagcaa cttcagggtg     960

cagccaacag agtccatcgt gcgctttccc aatatcacca acctgtgccc ttttggcgag    1020

gtgttcaatg ccacacgctt cgccagcgtg tacgcctgga ataggaagcg catctccaac    1080

tgcgtggccg actattctgt gctgtacaac agcgcctcct tctctacctt taagtgttat 1140

ggcgtgagcc ccaccaagct gaatgatctg tgctttacaa acgtgtacgc cgattccttc 1200

gtgatcaggg gcgacgaggt gcgccagatc gcaccaggac agaccggcaa gatcgcagac 1260

tacaattata agctgcctga cgatttcaca ggctgcgtga tcgcctggaa ctctaacaat 1320

ctggatagca aagtgggcgg caactacaat tatctgtacc ggctgtttag aaagtctaat 1380

ctgaagccat tcgagcggga catctccacc gagatctacc aggccggctc tacaccctgc 1440

aatggcgtgg agggctttaa ctgttatttc cctctgcagt cctacggctt ccagccaacc 1500

aacggcgtgg gctatcagcc ctacagagtg gtggtgctgt cttttgagct gctgcacgca 1560

cctgcaaccg tgtgcggccc aaagaagagc acaaatctgg tgaagaacaa gtgcgtgaac 1620

ttcaacttca acggactgac cggcacaggc gtgctgaccg agagcaacaa gaagttcctg 1680

ccatttcagc agttcggcag ggacatcgca gataccacag acgccgtgcg cgaccctcag 1740

accctggaga tcctggacat cacaccatgt tccttcggcg gcgtgtctgt gatcacccca 1800

ggcaccaata catccaacca ggtggccgtg ctgtatcagg acgtgaattg cacagaggtg 1860

cccgtggcaa tccacgcaga tcagctgacc cctacatggc gggtgtactc taccggcagc 1920

aacgtgttcc agacaagagc cggatgcctg atcggagcag agcacgtgaa caatagctat 1980

gagtgcgaca tccctatcgg cgccggcatc tgtgcctcct accagaccca gacaaactcc 2040

ccaaggagag cccggtctgt ggccagccag tccatcatcg cctataccat gagcctgggc 2100

gccgagaaca gcgtggccta ctccaacaat tctatcgcca tccctaccaa cttcacaatc 2160

agcgtgacca cagagatcct gccagtgagc atgaccaaga catccgtgga ctgcaccatg 2220

tatatctgtg gcgattccac agagtgttct aacctgctgc tgcagtacgg ctccttttgc 2280

acccagctga atagagccct gacaggcatc gccgtggagc aggacaagaa cacccaggag 2340

gtgttcgccc aggtgaagca gatctacaag acaccaccca tcaaggactt tggcggcttc 2400

aacttcagcc agatcctgcc cgatcctagc aagccatcca agcggtcttt tatcgaggac 2460

ctgctgttca acaaggtgac cctggccgat gccggcttca tcaagcagta tggcgattgt 2520

ctgggcgaca tcgccgccag agacctgatc tgcgcccaga agtttaatgg cctgaccgtg 2580

ctgcctccac tgctgacaga tgagatgatc gcacagtaca cctctgccct gctggccggc 2640

accatcacaa gcggatggac attcggcgca ggagccgccc tgcagatccc ctttgccatg 2700

cagatggcct atcggttcaa cggcatcggc gtgacccaga atgtgctgta cgagaaccag 2760

aagctgatcg ccaatcagtt taacagcgcc atcggcaaga tccaggactc tctgagctcc 2820

accgccagcg ccctgggcaa gctgcaggat gtggtgaatc agaacgccca ggccctgaat 2880

acactggtga agcagctgtc tagcaacttc ggcgccatct cctctgtgct gaatgacatc 2940

ctgagccggc tggacaaggt ggaggcagag gtgcagatcg accggctgat caccggcaga 3000

ctgcagtccc tgcagaccta cgtgacacag cagctgatca gggcagcaga gatcagggcc 3060

tctgccaatc tggccgccac aaagatgagc gagtgcgtgc tgggacagtc caagagggtg 3120

gacttttgcg gcaagggcta tcacctgatg agcttcccac agtccgcccc tcacggagtg 3180

gtgtttctgc acgtgaccta cgtgccagcc caggagaaga acttcaccac agcccccgcc 3240

atctgtcacg atggcaaggc ccactttcct agggagggcg tgttcgtgag caacggcacc 3300

cactggtttg tgacacagcg caatttctac gagccacaga tcatcaccac agacaatacc 3360

ttcgtgtccg gcaactgcga cgtggtcatc ggcatcgtga acaatacagt gtatgatcct 3420

ctgcagccag agctggactc ttttaaggag gagctggata agtacttcaa gaatcacacc 3480

agccccgacg tggatctggg cgacatctct ggcatcaatg ccagcgtggt gaacatccag 3540

aaggagatcg acagactgaa cgaggtggcc aagaatctga acgagagcct gatcgatctg 3600

caggagctgg gcaagtatga gcagtacatc aagtggccct ggtatatctg gctgggcttc 3660

atcgccggcc tgatcgccat cgtgatggtg accatcatgc tgtgctgtat gacaagctgc 3720

tgttcctgcc tgaagggctg ctgttcttgt ggcagctgct gtaagtttga tgaggacgat 3780

tccgagcctg tgctgaaggg cgtgaagctg cactacacct aa 3822

<210> 2
<211> 3825
<212> DNA
<213> Artificial Sequence

<220>
<223> CDS

<400> 2
atgtttgtct tcctggtgct gctgcctctg gtctcttctc agtgtgtgaa cctgaccacc 60

aggacacagc tgcctcctgc ttacaccaac tccttcacac ggggcgtcta ctaccctgac 120

aaggtcttca ggtcctctgt gctgcactcc acacaagacc tgttcctgcc attcttctcc 180

aacgtgacct ggttccatgc catccatgtc tctggcacca atggcaccaa gaggttcgac 240

aaccctgtgc tgccattcaa cgacggcgtc tactttgcct ccaccgagaa gtccaacatc 300

atcaggggct ggatctttgg caccaccctg gactccaaga cccagtccct gctgattgtg 360

aacaatgcca ccaacgtggt gatcaaagtc tgcgagttcc agttctgcaa tgacccattc 420

ctgggcgtct actaccacaa gaacaacaag tcctggatgg agtctgagtt cagggtctac 480

tcctctgcca acaactgcac ctttgaatat gtctcccagc cattcctgat ggacctggag 540

ggcaagcagg gcaacttcaa gaacctgagg gagttcgtct tcaagaacat cgacggctac 600

ttcaagatct actccaagca cacacccatt aatctcgtcc gggatctgcc tcagggcttc 660

tctgccctgg agcccctggt ggacctgccc attggcatca acatcacacg ctttcagacc 720

ctgctggctc tgcaccggtc ttacctgaca cctggcgact cctcctctgg ctggacagct 780

ggcgctgctg cctactatgt cggctacctg cagcctagga ccttcctgct gaagtacaat 840

gagaatggca ccatcacaga tgctgtggac tgtgccctgg accccctgtc tgagaccaag 900

tgcaccctga agtccttcac agtcgagaag ggcatctacc agacctccaa cttccgggtg 960

cagcccacag agtccattgt caggttcccc aacatcacca acctgtgccc atttggcgag 1020

gtcttcaatg ccacacgctt tgcctctgtc tatgcctgga acaggaagag gatctccaac 1080

tgtgtggctg actactctgt gctgtacaac tctgcctcct tctccacatt taagtgctat 1140

ggcgtctccc ccaccaagct gaatgacctg tgcttcacca atgtctatgc tgactccttt 1200

gtgatccggg gcgatgaagt taggcagatt gcccctggcc agacaggcaa gattgctgac 1260

tacaactaca agctgcctga tgacttcacc ggctgtgtga ttgcctggaa ctccaacaac 1320

ctggactcta aagttggcgg caactacaac tacctgtaca ggctgttcag gaagtccaac 1380

ctgaagccat ttgagaggga catctccaca gagatctacc aggctggctc caccccatgc 1440

aatggcgtgg agggcttcaa ctgctacttc cccctgcaat cctatggctt ccaacccacc 1500

aatggcgtgg gctaccagcc atacagggtg gtggtgctgt cctttgagct gctgcatgcc 1560

cctgccaccg tctgtggccc caagaagtcc accaacctcg tgaagaacaa atgtgtgaac 1620

ttcaacttca acggcctgac aggcacaggc gtgctgacag agtccaacaa gaagttcctg 1680

ccattccaac agtttggcag ggacattgct gacaccaccg atgctgtcag ggacccccag 1740

accctggaga tcctggacat caccccatgc tcctttggcg gcgtctctgt gatcacccct 1800

ggcaccaaca cctctaatca agtcgctgtg ctgtaccagg acgtgaactg cacagaggtt 1860

cctgtggcca tccatgctga ccagctgacc cccacctgga gggtctactc caccggctcc 1920

aatgtcttcc aaacacgggc tggctgcctg attggcgctg agcatgtgaa caactcctat 1980

gagtgtgaca tccccattgg cgctggcatc tgtgcctcct accaaaccca gaccaactcc 2040

cctcggaggg ctcgctctgt ggcttctcag tctatcattg cctacaccat gtccctgggc 2100

gctgagaact ctgtggccta ctccaacaac tccattgcca tccccaccaa cttcaccatc 2160

tctgtgacca ccgagatcct gcctgtctcc atgaccaaga cctctgtgga ctgcaccatg 2220

tacatctgtg gcgactccac agagtgctcc aacctgctgc tgcagtatgg ctccttctgc 2280

acccaactga acagggccct gacaggcatt gctgtggagc aggacaagaa cacccaggag 2340

gtctttgccc aagtcaagca gatctacaag acccccccca tcaaggactt tggcggcttc 2400

aacttctccc aaatcctgcc tgacccatcc aagccatcca agaggtcctt cattgaggac 2460

ctgctgttca acaaggttac cctggctgat gctggcttca tcaagcagta tggcgactgc 2520

ctgggcgaca ttgctgccag ggacctgatc tgtgcccaaa agttcaatgg cctgacagtg 2580

ctgccccccc tgctgaccga tgagatgatt gcccagtaca catctgctct gctggctggc 2640

acaatcacct ctggctggac ctttggcgct ggcgctgccc tgcaaatccc atttgccatg 2700

caaatggcct acaggttcaa tggcattggc gtgacccaga acgtgctgta tgagaaccag 2760

aagctgattg ccaaccagtt caactctgcc attggcaaga tccaggactc cctgtcctcc 2820

accgcctctg ccctgggcaa gctgcaggat gtggtgaacc agaatgccca ggccctgaac 2880

accctggtga agcagctgtc ctccaacttt ggcgccatct cctctgtgct gaatgacatc 2940

ctgtcccggc tggacaaggt ggaggctgag gtgcagattg acaggctgat cacaggcagg 3000

ctgcagtccc tgcaaaccta tgtgacccag cagctgatca gggctgctga gatcagggcc 3060

tctgccaacc tggctgccac caagatgtct gaatgtgtgc tgggccagtc caagagggtg 3120

gacttctgtg gcaagggcta ccatctgatg tccttccccc aatctgcccc ccatggcgtg 3180

gtcttcctgc atgtgaccta tgtgcctgct caggagaaga acttcaccac agcccctgcc 3240

atctgccatg atggcaaggc ccacttccct cgggagggcg tctttgtctc caatggcacc 3300

cactggtttg tgacccagag gaacttctat gagcctcaga tcatcaccac agacaacacc 3360

tttgtctctg gcaactgtga tgtggtgatt ggcattgtga acaacacagt ctatgacccc 3420

ctgcagcctg agctggactc cttcaaagag gagctggaca agtacttcaa gaaccacacc 3480

tcccctgatg tggacctggg cgacatctct ggcatcaatg cctctgtggt gaacatccag 3540

aaggagattg acaggctgaa tgaggtggcc aagaacctga atgagtccct gattgacctg 3600

caagagctgg gcaagtatga gcagtacatc aagtggccat ggtacatctg gctgggcttc 3660

attgctggcc tgattgccat tgtgatggtg accatcatgc tgtgctgcat gacctcctgc 3720

tgctcctgcc tgaaaggctg ctgctcctgt ggctcctgct gcaagtttga tgaggatgac 3780

tctgagcctg tgctgaaggg cgtgaagctg cactacacct aataa 3825

<210> 3
<211> 59
<212> DNA
<213> Artificial Sequence

<220>
<223> primer

<400> 3
gcgtttaaac ttaagcttgg taccgagctc ggatccgcca ccatgtttgt ttttcttgt 59

<210> 4
<211> 42
<212> DNA
<213> Artificial Sequence

<220>
<223> primer

<400> 4

agaatagggc cctctagact agtttatgtg taatgtaatt tg                    42

<210>  5
<211>  59
<212>  DNA
<213>  Artificial Sequence

<220>
<223>  primer

<400>  5
gcgtttaaac ttaagcttgg taccgagctc ggatccgcca ccatgttcgt gtttctggt      59

<210>  6
<211>  45
<212>  DNA
<213>  Artificial Sequence

<220>
<223>  primer

<400>  6
agaatagggc cctctagact agtttatcag gtgtagtgca gcttc                     45

<210>  7
<211>  59
<212>  DNA
<213>  Artificial Sequence

<220>
<223>  primer

<400>  7
gcgtttaaac ttaagcttgg taccgagctc ggatccgcca ccatgtttgt cttcctggt      59

<210>  8
<211>  42
<212>  DNA
<213>  Artificial Sequence

<220>
<223>  primer

<400>  8
agaatagggc cctctagact agtttaggtg tagtgcagct tc                        42

<210>  9
<211>  26
<212>  DNA
<213>  Artificial Sequence

<220>
<223>  artificial sequence

<400>  9
tctagagggc cctattctat agtgtc                                          26

<210>  10
<211>  46
<212>  DNA
<213>  Artificial Sequence

<220>
<223>  primer

<400>  10
ggatccgagc tcggtaccaa gcttaagttt aaacgctaga gtccgg                    46

## Description

### TECHNICAL FIELD

The present disclosure relates to an adenovirus-vectored vaccine for preventing SARS-CoV-2 infection.

### BACKGROUND

Novel Severe Acute Respiratory Syndrome Coronavirus 2 (SARS-CoV-2) has a latent period up to 24 days, and is highly infectious. Unlike the virus that caused Severe Acute Respiratory Syndrome (SARS), some cases infected with SARS-CoV-2 have infectivity during the latent period, while some SARS-CoV-2 carriers do not show any obvious symptoms. This increases the difficulty of epidemic prevention and control. As of 20 July 2020, there have been close to 15 million confirmed cases, including deaths of above 600,000, all over the world. So far there is no specific medicine available for COVID-19. To control the epidemic, most countries mandated quarantine and closed unnecessary facilities, which resulted in huge economic loss. Therefore, it has become the most urgent and major need to rapidly develop preventive vaccines that can improve herd-immunity level and block the transmission of the virus.

Currently, there is no specific antiviral medicine and preventive vaccine against this novel coronavirus, in China and other countries. Preventing and blocking the viral transmission are the keys to control the epidemic. It is known that vaccines have played an irreplaceable role in eliminating various infectious diseases. The published results obtained from the genome alignment of a dozen of SARS-CoV-2 viruses showed that the difference among the viruses is very small, and no mutation has been found. Therefore, the outbreak of new epidemic would be greatly contained, if SARS-CoV-2 vaccines are successfully developed.

Adenovirus is a commonly used vector in vaccine development and gene therapy, and is widely applied in biomedical field. The adenovirus has lower toxicity as compared with other vectors, such as bacteria- or other viruses-based vectors, and only causes mild flu-like symptoms. Ad5-empty vectors are replication-defective Ad5 vectors lacking the E1 and E3 genes, and carry no foreign gene. Gene therapy products using Ad5 as carriers have been proved to be safe in many clinical trials.

SARS-CoV-2 has a complete genome as shown by NC_045512.2, in which the sequence of nucleic acids 21563 to 25384 is a coding sequence for Spike protein (S). The S protein is a major structural protein of a virion, and plays an important role in mediating the binding of the virion to a host cell receptor and inducing neutralizing antibodies. Vaccines, which use the S protein as an antigen, comprise nucleic acid-, subunit- and virus-vectored vaccines, and the efficacy of such vaccines are determined by the expression level and protein structure of the S protein.

However, it was shown by experiments that the gene coding for the Spike protein of natural SARS-CoV-2 has low expression level in human body. If a vaccine adopts natural genetic codons to express the S protein as the antigen, this vaccine would not produce sufficient antigen, and thus would be ineffective or have low titer, thereby being unable to fight the virus infection.

### SUMMARY

The present disclosure is intended to overcome the above shortcomings of the prior art, and provide an optimized nucleotide sequence for coding the S protein. As the optimized nucleotide sequence is integrated into 1) a DNA plasmid or 2) an adenovirus vector, the S protein can be produced at a higher level after the plasmid or adenovirus vector is transfected into human cells. Therefore, the optimized nucleotide sequence for coding the S protein is expected to be used as an antigen gene for a nucleic acid vaccine or a recombinant viral vaccine to prevent SARS-CoV-2 infection.

The technical solutions adopted by the present disclosure are as follows.

In a first aspect of the present disclosure, provided is a vaccine for preventing SARS-CoV-2 infection, wherein the vaccine comprises:
a) a nucleotide sequence as shown by SEQ ID NO: 1; or
b) a nucleotide sequence having at least 80%, 85%, 90%, 95% or 100% homology with the nucleotide as shown by SEQ ID NO: 1.

In some embodiments, said nucleotide sequence can express a protein in a human-derived cell or human body.

In some embodiments, in the human body, said protein is capable of:
inducing an immune response;
generating a molecular biology reporter;
generating a trace molecule for detection;
regulating a gene function; or
functioning as a therapeutic molecule.

The induced immune response may include antibody- and cell-mediated immune responses.

In some embodiments, the vaccine may further include a pharmaceutically-acceptable adjuvant, carrier, diluent or excipient.

In some embodiments, the vaccine may further include at least one medicament having therapeutic effect on coronavirus disease 2019 (COVID-19).

In a second aspect of the present disclosure, provided is an expression vector which comprises the nucleotide sequence according to the first aspect of the present disclosure.

In some embodiments, the nucleotide sequence according to the first aspect of the present disclosure may have a transcription direction opposite to those of the other genes in the vector. This can further ensure that the expressed S protein has a higher level of purity.

In some embodiments, the vector may be a DNA plasmid, an RNA expression plasmid, or a viral vector.

In some embodiments, the viral vector may be an adenovirus vector. In particular, the adenovirus vector may be a replication-defective Ad5 vector.

In a third aspect of the present disclosure, provided is use of the vaccine according to the first aspect of the present disclosure, which comprises:
preparing a COVID-19 detection reagent; and
preparing a gene-function regulator.

In a fourth aspect of the present disclosure, provided is a method for preventing or treating COVID-19 infection, which comprises administering a prophylactically or therapeutically effective amount of the vaccine according to the first aspect of the present disclosure to a human.

The beneficial effects of the present disclosure are as follows.

The vaccine according to some embodiments of the present disclosure has an S protein-coding nucleotide sequence, which is easily expressed in human cells and can induce the production of the S protein at a higher level. Such vaccine is expected to be used as a recombinant viral vaccine for preventing SARS-CoV-2 infection.

The vaccine according to some embodiments of the present disclosure has better safety.

As shown by the animal experiments performed in some examples of the present disclosure, the vaccine can produce a high titer of antibodies and cellular immunity after immunizing mice and macaque monkeys (*Macaca mulatta*)*.* The macaque monkeys were immunized with the vaccine through single intramuscular (I.M.) or intra-nasal/oral (I.N.) administration, and were challenged with the SARS-Cov-2 virus 30 days after the immunization. The results showed that the immunized macaque monkeys were fully resistant to the virus infection. When the macaque monkeys were intramuscularly immunized once with 1/10 dose (1×10¹⁰ vp/animal) of the vaccine and challenged with the SARS-Cov-2 virus 8 weeks after the immunization, the immunized macaque monkeys were also fully resistant to the virus infection. For the vaccinated macaque monkeys, no lung damage caused by antibody-dependent enhancement (ADE) or the virus was observed after receiving the virus challenge. For most of the vaccinated macaque monkeys, the antibody level did not increase after receiving the virus challenge. It indicates that the virus was eliminated quickly, resulting in no stimulation effect on the immune system. Different from the whole-virus inactivated vaccine, the vaccine of the present disclosure, in addition to stimulating humoral immunity, also induces cellular immunity, which can further improve the protective effect on body function. Furthermore, since the intra-nasal/oral immunization also obtained good protective effect, it suggests the feasibility of mucosal- and non-injection immunization.

### BRIEF DESCRIPTION OF THE DRAWINGS

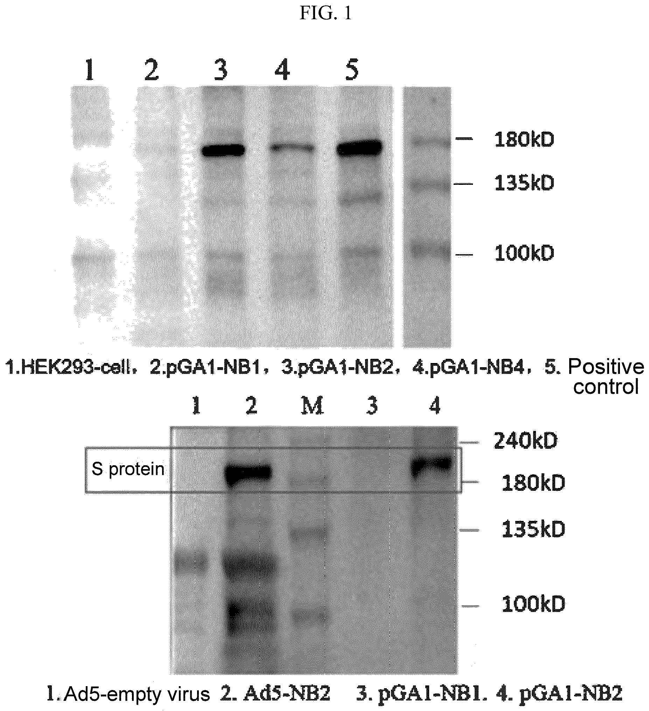

FIG. 1 shows the expression of S protein after transfecting human-derived cells with a plasmid containing the codon-optimized nucleotide sequence of the S gene.

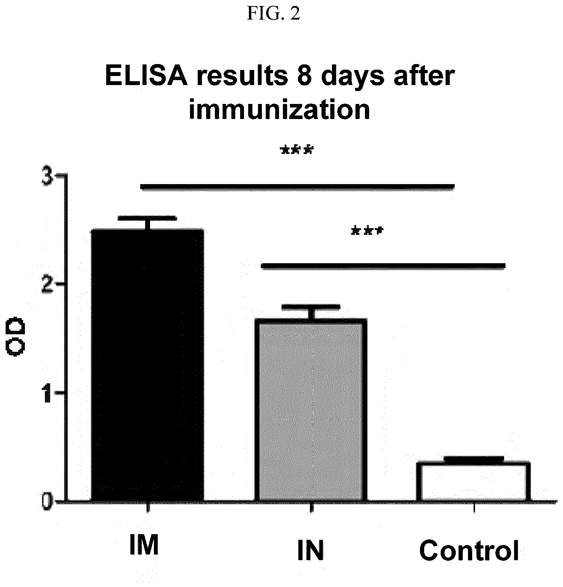

FIG. 2 shows the experimental results of animal immunogenicity evaluation, which illustrates that Ad5-NB2 can induce the production of the antibody in mice.

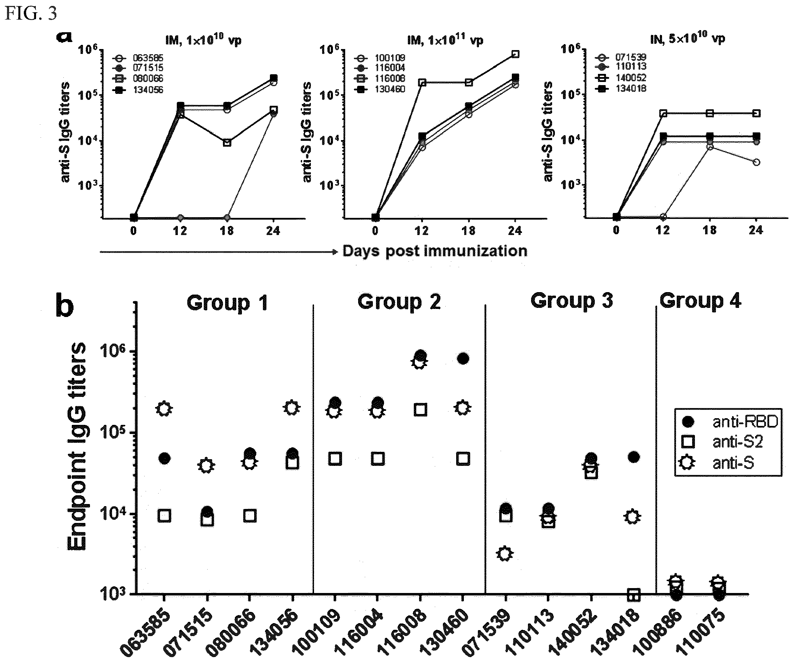

FIG. 3 shows the detection results of the binding antibody in serum of the macaque monkeys at different times after immunization, in which a: the detection results of the binding antibodies in serum of the macaque monkeys at different times after immunization; and b: the detection results of the binding antibodies in serum of the macaque monkeys 18 days after immunization.

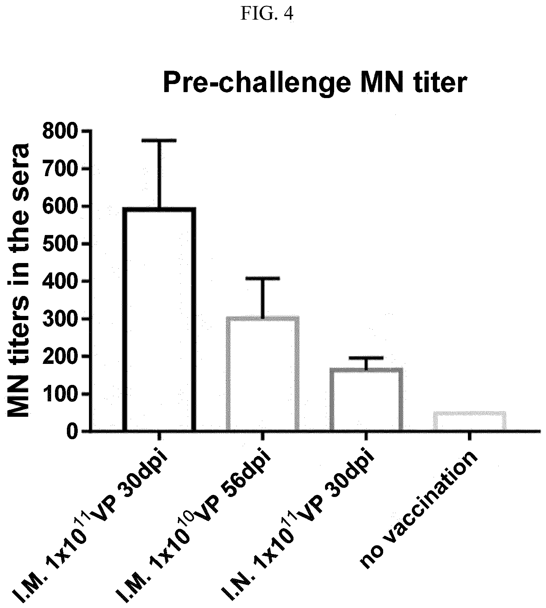

FIG. 4 shows the titer of neutralizing antibodies in serum of the immunized macaque monkeys against live novel coronaviruses.

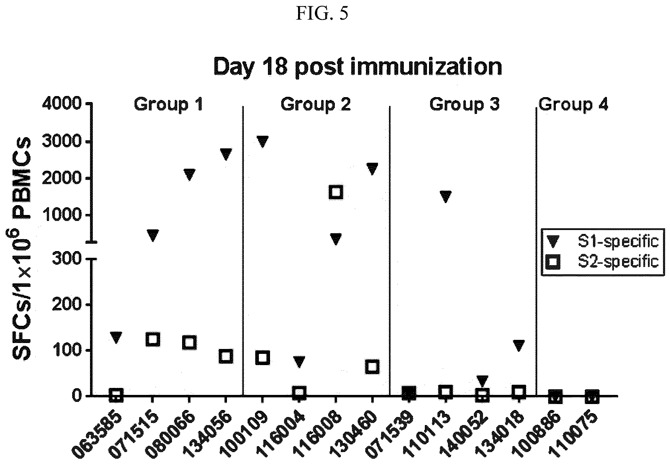

FIG. 5 shows the ELISpot results of the peripheral blood mononuclear cell (PBMCs) of the m*acaque monkeys* 18 days after immunization.

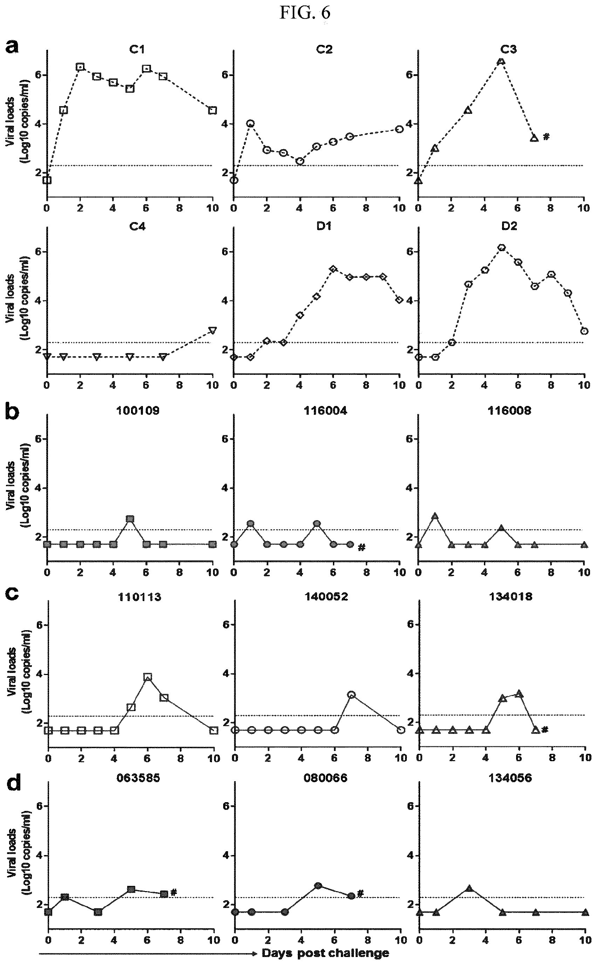

FIG. 6 shows the copy number of virus genome from the pharyngeal swabs of the macaque monkeys after virus challenge, in which a: the copy number changes of the virus genome in a control group after virus challenge; b: the copy number changes of the virus genome in an intramuscular injecting high-dose group after virus challenge; c: the copy number changes of the virus genome in an intramuscular injecting low-dose group after virus challenge; and d: the copy number changes of the virus genome in an intra-nasal/oral group after virus challenge.

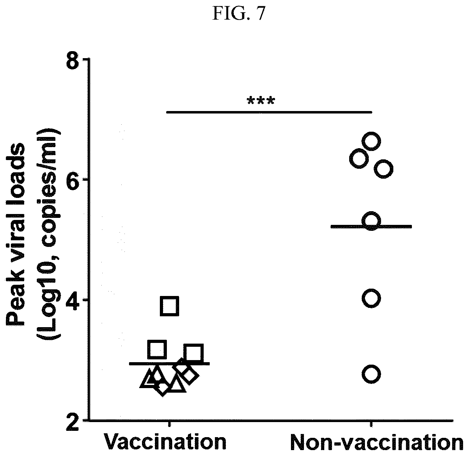

FIG. 7 shows the viral load peaks from the pharyngeal swabs of vaccinated and non-vaccinated macaque monkeys, in which each circle represents the viral load peak of a macaque monkey; each diamond represents a macaque monkey intramuscularly vaccinated with a high dose vaccine; each square represents a macaque monkey vaccinated through intra-nasal/oral administration; each triangle represents a macaque monkey intramuscularly vaccinated with a low dose vaccine; and each black circle represents a non-vaccinated macaque monkey.

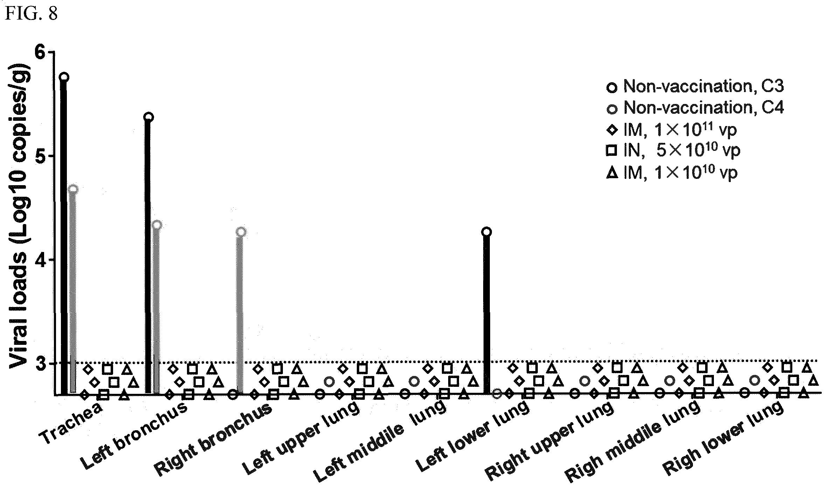

FIG. 8 shows the copy number of virus genome in different tissues of macaque monkeys after virus challenge.

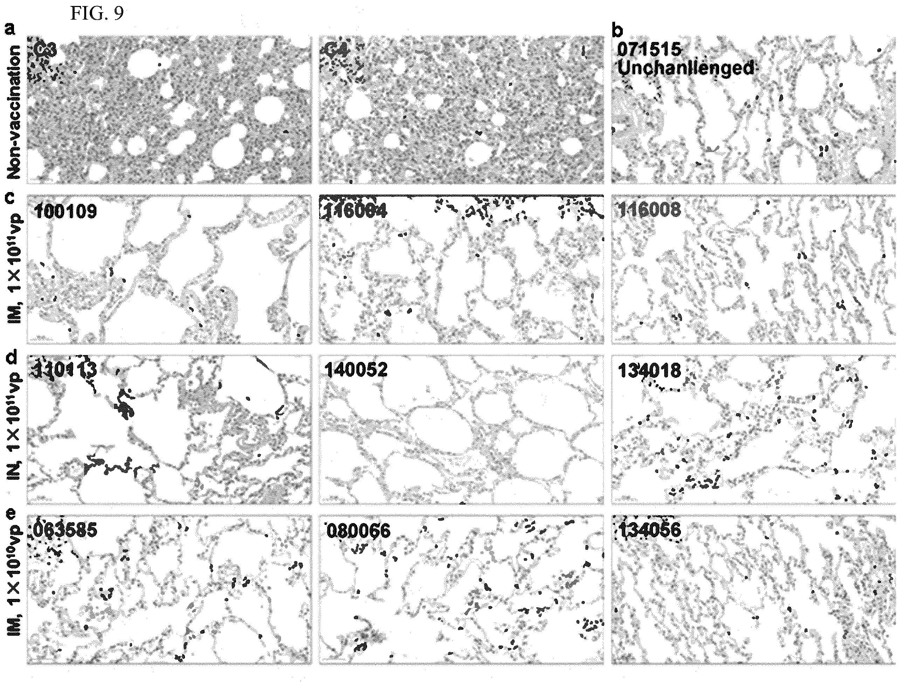

FIG. 9 shows the pathological results of the lung of the macaque monkeys after virus challenge, in which a: the pathological sections of the lung of the macaque monkeys from the control group after virus challenge (HE staining); b: the pathological sections of the lung of the macaque monkeys from the control group without virus challenge (HE staining); c: the pathological sections of the lung of the macaque monkeys from the high-dose intramuscularly administrated group (1×10¹¹ VP) after virus challenge (HE staining); d: the pathological sections of the lung of the macaque monkeys from the intra-nasally/orally immunized group (5×10¹⁰ vp/route) after virus challenge (HE staining); and e: the pathological sections of the lung of the macaque monkeys from the low-dose intramuscularly administrated group (1×10¹⁰ vp) after virus challenge (HE staining).

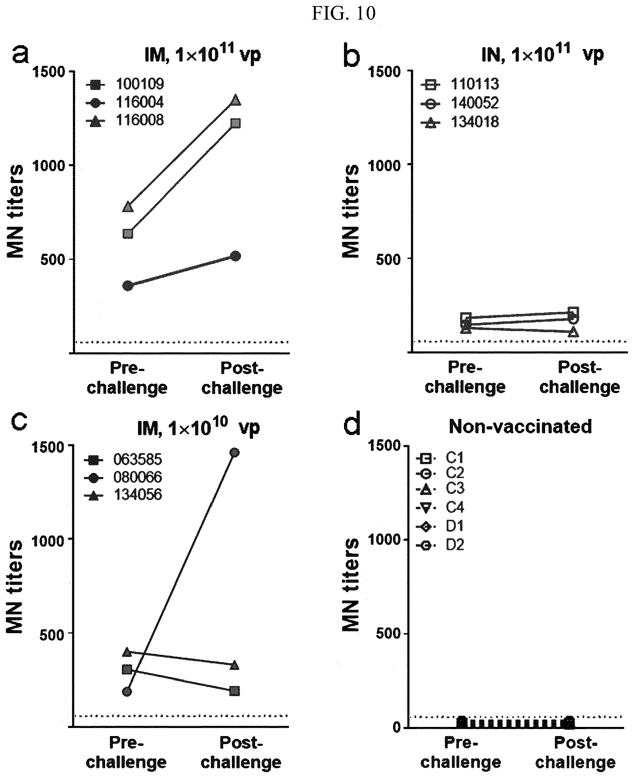

FIG. 10 shows the changes of the anti-SARS-CoV-2 neutralizing antibodies of the macaque monkeys before and after virus challenge, in which a: the changes of the anti-SARS-CoV-2 neutralizing antibodies of the intramuscular administration high-dose group before and after virus challenge; b: the changes of the anti-SARS-CoV-2 neutralizing antibodies of the intra-nasal/oral administration group before and after virus challenge; c: the changes of the anti-SARS-CoV-2 neutralizing antibodies of the intramuscular administration low-dose group before and after virus challenge; and d: the changes of the anti-SARS-CoV-2 neutralizing antibodies of the control group before and after virus challenge.

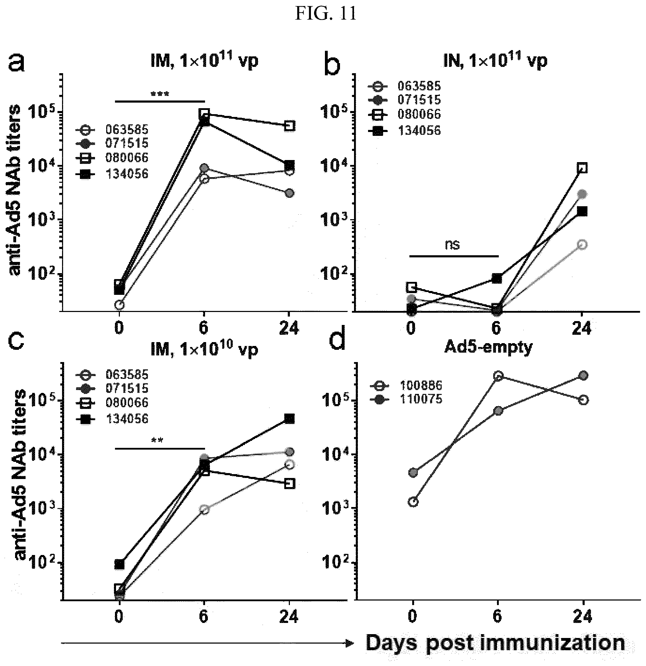

FIG. 11 shows the changes of the anti-Ad5 neutralizing antibodies of the macaque monkeys before and after immunization, in which a: the changes of the anti-Ad5 neutralizing antibodies of the intramuscular administration high-dose group before and after immunization; b: the changes of the anti-Ad5 neutralizing antibodies of the intramuscular administration low-dose group before and after immunization; c: the changes of the anti-Ad5 neutralizing antibodies of the intra-nasal/oral administration group before and after immunization; and d: the changes of the anti-Ad5 neutralizing antibodies of the control group before and after immunization.

### DETAILED DESCRIPTION

The Spike protein (S) of SARS-CoV-2 has an amino acid sequence as shown by YP_009724390.1, designated as NB1.

Precursor mRNA in eukaryotic cells can produce various mRNA splice variants through different splicing manners (by selecting different combination of splice sites), eventually leading to different proteins produced from the same gene sequence. This is very unfavorable for the expression of a protein. The inventor performed codon optimization to the wild-type natural nucleotide sequence and removed potential variable cleavage sites based on self-owned technology, thereby ensuring the uniqueness of expressed protein and reducing the difficulty in subsequent protein purification. The optimized nucleotide sequence is designated as NB2, and is as shown by SEQ ID NO: 1:

ATGTTCGTGTTTCTGGTGCTGCTGCCTCTGGTGAGCTCCCAGTGCGTGAACCTG
ACCACAAGGACCCAGCTGCCACCTGCCTATACCAATAGCTTCACACGGGGCGTGTAC
TATCCCGACAAGGTGTTTAGATCTAGCGTGCTGCACTCCACCCAGGATCTGTTTCTGC
CTTTCTTTTCTAACGTGACATGGTTCCACGCCATCCACGTGTCCGGCACCAATGGCA
CAAAGCGGTTCGACAATCCAGTGCTGCCCTTTAACGATGGCGTGTACTTCGCCTCCA
CCGAGAAGTCTAACATCATCAGAGGCTGGATCTTTGGCACCACACTGGACAGCAAG
ACCCAGTCCCTGCTGATCGTGAACAATGCCACAAACGTGGTCATCAAGGTGTGCGA
GTTCCAGTTTTGTAATGATCCCTTCCTGGGCGTGTACTATCACAAGAACAATAAGTCT
TGGATGGAGAGCGAGTTTAGGGTGTATTCCTCTGCCAACAATTGCACCTTTGAGTAC
GTGAGCCAGCCTTTCCTGATGGACCTGGAGGGCAAGCAGGGCAATTTCAAGAACCT

GAGGGAGTTCGTGTTTAAGAATATCGATGGCTACTTCAAGATCTACTCCAAGCACAC
ACCAATCAACCTGGTGCGCGACCTGCCACAGGGCTTCTCTGCCCTGGAGCCACTGG
TGGATCTGCCCATCGGCATCAACATCACCCGGTTTCAGACACTGCTGGCCCTGCACA
GAAGCTACCTGACCCCAGGCGACAGCTCCTCTGGATGGACAGCAGGAGCTGCCGCC
TACTATGTGGGCTATCTGCAGCCCCGCACCTTCCTGCTGAAGTACAACGAGAATGGC
ACCATCACAGACGCAGTGGATTGCGCCCTGGACCCCCTGTCTGAGACCAAGTGTAC
ACTGAAGAGCTTTACAGTGGAGAAGGGCATCTACCAGACCAGCAACTTCAGGGTGC
AGCCAACAGAGTCCATCGTGCGCTTTCCCAATATCACCAACCTGTGCCCTTTTGGCG
AGGTGTTCAATGCCACACGCTTCGCCAGCGTGTACGCCTGGAATAGGAAGCGCATCT
CCAACTGCGTGGCCGACTATTCTGTGCTGTACAACAGCGCCTCCTTCTCTACCTTTAA
GTGTTATGGCGTGAGCCCCACCAAGCTGAATGATCTGTGCTTTACAAACGTGTACGC
CGATTCCTTCGTGATCAGGGGCGACGAGGTGCGCCAGATCGCACCAGGACAGACCG
GCAAGATCGCAGACTACAATTATAAGCTGCCTGACGATTTCACAGGCTGCGTGATCG
CCTGGAACTCTAACAATCTGGATAGCAAAGTGGGCGGCAACTACAATTATCTGTACC
GGCTGTTTAGAAAGTCTAATCTGAAGCCATTCGAGCGGGACATCTCCACCGAGATCT
ACCAGGCCGGCTCTACACCCTGCAATGGCGTGGAGGGCTTTAACTGTTATTTCCCTC
TGCAGTCCTACGGCTTCCAGCCAACCAACGGCGTGGGCTATCAGCCCTACAGAGTG
GTGGTGCTGTCTTTTGAGCTGCTGCACGCACCTGCAACCGTGTGCGGCCCAAAGAA
GAGCACAAATCTGGTGAAGAACAAGTGCGTGAACTTCAACTTCAACGGACTGACCG
GCACAGGCGTGCTGACCGAGAGCAACAAGAAGTTCCTGCCATTTCAGCAGTTCGGC
AGGGACATCGCAGATACCACAGACGCCGTGCGCGACCCTCAGACCCTGGAGATCCT
GGACATCACACCATGTTCCTTCGGCGGCGTGTCTGTGATCACCCCAGGCACCAATAC
ATCCAACCAGGTGGCCGTGCTGTATCAGGACGTGAATTGCACAGAGGTGCCCGTGG
CAATCCACGCAGATCAGCTGACCCCTACATGGCGGGTGTACTCTACCGGCAGCAACG
TGTTCCAGACAAGAGCCGGATGCCTGATCGGAGCAGAGCACGTGAACAATAGCTAT
GAGTGCGACATCCCTATCGGCGCCGGCATCTGTGCCTCCTACCAGACCCAGACAAAC
TCCCCAAGGAGAGCCCGGTCTGTGGCCAGCCAGTCCATCATCGCCTATACCATGAGC
CTGGGCGCCGAGAACAGCGTGGCCTACTCCAACAATTCTATCGCCATCCCTACCAAC
TTCACAATCAGCGTGACCACAGAGATCCTGCCAGTGAGCATGACCAAGACATCCGT
GGACTGCACCATGTATATCTGTGGCGATTCCACAGAGTGTTCTAACCTGCTGCTGCA
GTACGGCTCCTTTTGCACCCAGCTGAATAGAGCCCTGACAGGCATCGCCGTGGAGC
AGGACAAGAACACCCAGGAGGTGTTCGCCCAGGTGAAGCAGATCTACAAGACACC
ACCCATCAAGGACTTTGGCGGCTTCAACTTCAGCCAGATCCTGCCCGATCCTAGCAA

GCCATCCAAGCGGTCTTTATCGAGGACCTGCTGTTCAACAAGGTGACCCTGGCCGA
TGCCGGCTTCATCAAGCAGTATGGCGATTGTCTGGGCGACATCGCCGCCAGAGACCT
GATCTGCGCCCAGAAGTTTAATGGCCTGACCGTGCTGCCTCCACTGCTGACAGATGA
GATGATCGCACAGTACACCTCTGCCCTGCTGGCCGGCACCATCACAAGCGGATGGA
CATTCGGCGCAGGAGCCGCCCTGCAGATCCCCTTTGCCATGCAGATGGCCTATCGGT
TCAACGGCATCGGCGTGACCCAGAATGTGCTGTACGAGAACCAGAAGCTGATCGCC
AATCAGTTTAACAGCGCCATCGGCAAGATCCAGGACTCTCTGAGCTCCACCGCCAG
CGCCCTGGGCAAGCTGCAGGATGTGGTGAATCAGAACGCCCAGGCCCTGAATACAC
TGGTGAAGCAGCTGTCTAGCAACTTCGGCGCCATCTCCTCTGTGCTGAATGACATCC
TGAGCCGGCTGGACAAGGTGGAGGCAGAGGTGCAGATCGACCGGCTGATCACCGG
CAGACTGCAGTCCCTGCAGACCTACGTGACACAGCAGCTGATCAGGGCAGCAGAGA
TCAGGGCCTCTGCCAATCTGGCCGCCACAAAGATGAGCGAGTGCGTGCTGGGACAG
TCCAAGAGGGTGGACTTTTGCGGCAAGGGCTATCACCTGATGAGCTTCCCACAGTC
CGCCCCTCACGGAGTGGTGTTTCTGCACGTGACCTACGTGCCAGCCCAGGAGAAGA
ACTTCACCACAGCCCCCGCCATCTGTCACGATGGCAAGGCCCACTTTCCTAGGGAG
GGCGTGTTCGTGAGCAACGGCACCCACTGGTTTGTGACACAGCGCAATTTCTACGA
GCCACAGATCATCACCACAGACAATACCTTCGTGTCCGGCAACTGCGACGTGGTCAT
CGGCATCGTGAACAATACAGTGTATGATCCTCTGCAGCCAGAGCTGGACTCTTTTAA
GGAGGAGCTGGATAAGTACTTCAAGAATCACACCAGCCCCGACGTGGATCTGGGCG
ACATCTCTGGCATCAATGCCAGCGTGGTGAACATCCAGAAGGAGATCGACAGACTG
AACGAGGTGGCCAAGAATCTGAACGAGAGCCTGATCGATCTGCAGGAGCTGGGCA
AGTATGAGCAGTACATCAAGTGGCCCTGGTATATCTGGCTGGGCTTCATCGCCGGCC
TGATCGCCATCGTGATGGTGACCATCATGCTGTGCTGTATGACAAGCTGCTGTTCCTG
CCTGAAGGGCTGCTGTTCTTGTGGCAGCTGCTGTAAGTTTGATGAGGACGATTCCGA
GCCTGTGCTGAAGGGCGTGAAGCTGCACTACACCTAA (SEQ ID NO.: 1).

In order to reduce the GC-content of the gene, the inventor further optimized the codons based on NB2, resulting in a sequence designated as NB4. NB4 has a sequence as shown by SEQ ID No.: 2 below, and has 85.93% sequence identity with that of NB2. 
ATGTTTGTCTTCCTGGTGCTGCTGCCTCTGGTCTCTTCTCAGTGTGTGAACCTGA
CCACCAGGACACAGCTGCCTCCTGCTTACACCAACTCCTTCACACGGGGCGTCTACT
ACCCTGACAAGGTCTTCAGGTCCTCTGTGCTGCACTCCACACAAGACCTGTTCCTGC
CATTCTTCTCCAACGTGACCTGGTTCCATGCCATCCATGTCTCTGGCACCAATGGCAC
 
CAAGAGGTTCGACAACCCTGTGCTGCCATTCAACGACGGCGTCTACTTTGCCTCCAC
CGAGAAGTCCAACATCATCAGGGGCTGGATCTTTGGCACCACCCTGGACTCCAAGA
CCCAGTCCCTGCTGATTGTGAACAATGCCACCAACGTGGTGATCAAAGTCTGCGAGT
TCCAGTTCTGCAATGACCCATTCCTGGGCGTCTACTACCACAAGAACAACAAGTCCT
GGATGGAGTCTGAGTTCAGGGTCTACTCCTCTGCCAACAACTGCACCTTTGAATATG
TCTCCCAGCCATTCCTGATGGACCTGGAGGGCAAGCAGGGCAACTTCAAGAACCTG
AGGGAGTTCGTCTTCAAGAACATCGACGGCTACTTCAAGATCTACTCCAAGCACAC
ACCCATTAATCTCGTCCGGGATCTGCCTCAGGGCTTCTCTGCCCTGGAGCCCCTGGT
GGACCTGCCCATTGGCATCAACATCACACGCTTTCAGACCCTGCTGGCTCTGCACCG
GTCTTACCTGACACCTGGCGACTCCTCCTCTGGCTGGACAGCTGGCGCTGCTGCCTA
CTATGTCGGCTACCTGCAGCCTAGGACCTTCCTGCTGAAGTACAATGAGAATGGCAC
CATCACAGATGCTGTGGACTGTGCCCTGGACCCCCTGTCTGAGACCAAGTGCACCCT
GAAGTCCTTCACAGTCGAGAAGGGCATCTACCAGACCTCCAACTTCCGGGTGCAGC
CCACAGAGTCCATTGTCAGGTTCCCCAACATCACCAACCTGTGCCCATTTGGCGAGG
TCTTCAATGCCACACGCTTTGCCTCTGTCTATGCCTGGAACAGGAAGAGGATCTCCA
ACTGTGTGGCTGACTACTCTGTGCTGTACAACTCTGCCTCCTTCTCCACATTTAAGTG
CTATGGCGTCTCCCCCACCAAGCTGAATGACCTGTGCTTCACCAATGTCTATGCTGA
CTCCTTTGTGATCCGGGGCGATGAAGTTAGGCAGATTGCCCCTGGCCAGACAGGCA
AGATTGCTGACTACAACTACAAGCTGCCTGATGACTTCACCGGCTGTGTGATTGCCT
GGAACTCCAACAACCTGGACTCTAAAGTTGGCGGCAACTACAACTACCTGTACAGG
CTGTTCAGGAAGTCCAACCTGAAGCCATTTGAGAGGGACATCTCCACAGAGATCTA
CCAGGCTGGCTCCACCCCATGCAATGGCGTGGAGGGCTTCAACTGCTACTTCCCCCT
GCAATCCTATGGCTTCCAACCCACCAATGGCGTGGGCTACCAGCCATACAGGGTGGT
GGTGCTGTCCTTTGAGCTGCTGCATGCCCCTGCCACCGTCTGTGGCCCCAAGAAGTC
CACCAACCTCGTGAAGAACAAATGTGTGAACTTCAACTTCAACGGCCTGACAGGCA
CAGGCGTGCTGACAGAGTCCAACAAGAAGTTCCTGCCATTCCAACAGTTTGGCAGG
GACATTGCTGACACCACCGATGCTGTCAGGGACCCCCAGACCCTGGAGATCCTGGA
CATCACCCCATGCTCCTTTGGCGGCGTCTCTGTGATCACCCCTGGCACCAACACCTC
TAATCAAGTCGCTGTGCTGTACCAGGACGTGAACTGCACAGAGGTTCCTGTGGCCAT
CCATGCTGACCAGCTGACCCCCACCTGGAGGGTCTACTCCACCGGCTCCAATGTCTT
CCAAACACGGGCTGGCTGCCTGATTGGCGCTGAGCATGTGAACAACTCCTATGAGT
GTGACATCCCCATTGGCGCTGGCATCTGTGCCTCCTACCAAACCCAGACCAACTCCC
CTCGGAGGGCTCGCTCTGTGGCTTCTCAGTCTATCATTGCCTACACCATGTCCCTGGG
 
CGCTGAGAACTCTGTGGCCTACTCCAACAACTCCATTGCCATCCCCACCAACTTCAC
CATCTCTGTGACCACCGAGATCCTGCCTGTCTCCATGACCAAGACCTCTGTGGACTG
CACCATGTACATCTGTGGCGACTCCACAGAGTGCTCCAACCTGCTGCTGCAGTATGG
CTCCTTCTGCACCCAACTGAACAGGGCCCTGACAGGCATTGCTGTGGAGCAGGACA
AGAACACCCAGGAGGTCTTTGCCCAAGTCAAGCAGATCTACAAGACCCCCCCCATC
AAGGACTTTGGCGGCTTCAACTTCTCCCAAATCCTGCCTGACCCATCCAAGCCATCC
AAGAGGTCCTTCATTGAGGACCTGCTGTTCAACAAGGTTACCCTGGCTGATGCTGGC
TTCATCAAGCAGTATGGCGACTGCCTGGGCGACATTGCTGCCAGGGACCTGATCTGT
GCCCAAAAGTTCAATGGCCTGACAGTGCTGCCCCCCCTGCTGACCGATGAGATGATT
GCCCAGTACACATCTGCTCTGCTGGCTGGCACAATCACCTCTGGCTGGACCTTTGGC
GCTGGCGCTGCCCTGCAAATCCCATTTGCCATGCAAATGGCCTACAGGTTCAATGGC
ATTGGCGTGACCCAGAACGTGCTGTATGAGAACCAGAAGCTGATTGCCAACCAGTT
CAACTCTGCCATTGGCAAGATCCAGGACTCCCTGTCCTCCACCGCCTCTGCCCTGGG
CAAGCTGCAGGATGTGGTGAACCAGAATGCCCAGGCCCTGAACACCCTGGTGAAGC
AGCTGTCCTCCAACTTTGGCGCCATCTCCTCTGTGCTGAATGACATCCTGTCCCGGCT
GGACAAGGTGGAGGCTGAGGTGCAGATTGACAGGCTGATCACAGGCAGGCTGCAG
TCCCTGCAAACCTATGTGACCCAGCAGCTGATCAGGGCTGCTGAGATCAGGGCCTCT
GCCAACCTGGCTGCCACCAAGATGTCTGAATGTGTGCTGGGCCAGTCCAAGAGGGT
GGACTTCTGTGGCAAGGGCTACCATCTGATGTCCTTCCCCCAATCTGCCCCCCATGG
CGTGGTCTTCCTGCATGTGACCTATGTGCCTGCTCAGGAGAAGAACTTCACCACAGC
CCCTGCCATCTGCCATGATGGCAAGGCCCACTTCCCTCGGGAGGGCGTCTTTGTCTC
CAATGGCACCCACTGGTTTGTGACCCAGAGGAACTTCTATGAGCCTCAGATCATCAC
CACAGACAACACCTTTGTCTCTGGCAACTGTGATGTGGTGATTGGCATTGTGAACAA
CACAGTCTATGACCCCCTGCAGCCTGAGCTGGACTCCTTCAAAGAGGAGCTGGACA
AGTACTTCAAGAACCACACCTCCCCTGATGTGGACCTGGGCGACATCTCTGGCATCA
ATGCCTCTGTGGTGAACATCCAGAAGGAGATTGACAGGCTGAATGAGGTGGCCAAG
AACCTGAATGAGTCCCTGATTGACCTGCAAGAGCTGGGCAAGTATGAGCAGTACAT
CAAGTGGCCATGGTACATCTGGCTGGGCTTCATTGCTGGCCTGATTGCCATTGTGATG
GTGACCATCATGCTGTGCTGCATGACCTCCTGCTGCTCCTGCCTGAAAGGCTGCTGC
TCCTGTGGCTCCTGCTGCAAGTTTGATGAGGATGACTCTGAGCCTGTGCTGAAGGGC
GTGAAGCTGCACTACACCTAATAA (SEQ ID NO: 2)
 **Construction of a vector for the expression of the Spike gene:**

Using NB1, NB2 and NB4 as templates, respectively, the NB1 fragment was obtained through PCR amplification with primers NB1-F and NB1-R, the NB2 fragment was obtained through PCR amplification with primers NB2-F and NB2-R, and the NB4 fragment was obtained through PCR amplification with primers NB4-F and NB4-R. The vector plasmid backbone pGA1 was PCR amplified, by using CMV-R and BGH-F as primers, and using a pGA1-EGFP plasmid, which was owned by our company, as the template. Then, two-fragment *in vitro* recombination was carried out by using a homologous recombination enzyme (Exnase) with the NB1, NB2 or NB4 fragment, respectively, to obtain pGA1-NB1, pGA1-NB2 and pGA1-NB4. The pGA1-NB2 was linearized by using Bstz17I and SgarAI. Then, homologous recombination was carried out between the pGA1-NB2 and pAd5ΔE1ΔE3 (i.e., Ad5 empty vector), which was digested to remove the unique restriction site in the E1 region and linearized, by using competent BJ5183 cells to obtain a pAd5-NB2 vaccine vector.

The primer sequences for amplifying NB1:
NB1-F: 
GCGTTTAAACTTAAGCTTGGTACCGAGCTCGGATCCGCCACCATGTTTGTTTTTCTTG
T (SEQ ID NO.: 3)
NB1-R: AGAATAGGGCCCTCTAGACTAGTTTATGTGTAATGTAATTTG (SEQ ID NO.: 4)

The primer sequences for amplifying NB2:
NB2-F: 
GCGTTTAAACTTAAGCTTGGTACCGAGCTCGGATCCGCCACCATGTTCGTGTTTCTG
GT (SEQ ID NO.: 5)
NB2-R: AGAATAGGGCCCTCTAGACTAGTTTATCAGGTGTAGTGCAGCTTC (SEQ ID NO.: 6)

The primer sequences for amplifying NB4:
NB4-F: 
GCGTTTAAACTTAAGCTTGGTACCGAGCTCGGATCCGCCACCATGTTTGTCTTCCTG
GT (SEQ ID NO.: 7)
NB4-R: AGAATAGGGCCCTCTAGACTAGTTTAGGTGTAGTGCAGCTTC (SEQ ID NO.: 8)

The primer sequences for amplifying pGA1:
BGH-F: TCTAGAGGGCCCTATTCTATAGTGTC (SEQ ID NO.: 9)
CMV-R: GGATCCGAGCTCGGTACCAAGCTTAAGTTTAAACGCTAGAGTCCGG (SEQ ID NO.: 10)
PCR conditions: 95°C for 3 min; 95°C for 30 s; 60°C for 30 s; 72°C for 2 min; 30 cycles; and 72°C for 5 min.

### Rescue and production of Ad5-NB2 vector

1) According to a conventional method, linearizing the pAd5-NB2 vector with AsiSI, purifying by precipitation in ethanol, and transfecting into 293 cells with cationic lipofection; 8 hours after the transfection, adding 2 ml of DMEM medium containing 5% fetal bovine serum, and incubating for 7-10 days to observe cytopathic effect (CPE).
2) As observing CPE, collecting the cells and culture supernatant, repeating a freeze-thaw step in water bath at 37°C and liquid nitrogen for 3 times, centrifuging to remove cell debris, and then infecting a 10 cm-dish with the supernatant;
3) After 2-3 days, collecting the cells and culture supernatant, repeating the freeze-thaw step for 3 times, centrifuging to remove cell debris, and then infecting 3-5 of 15 cm-dishes with the supernatant;
4) After 2-3 days, collecting the cells, repeating the freeze-thaw step for 3 times, centrifuging to remove cell debris, and then infecting 30 of 15 cm-dishes with the supernatant. After 2-3 days, collecting the cells, repeating the freeze-thaw step for 3 times, and centrifuging to remove cell debris.
5) Adding the supernatant into a cesium chloride density gradient centrifuge tube, and centrifuging at 4°C, 40000 rpm, for 4 hours;
6) Pipetting out the virus band, desalting, and subpackaging.

The virion titers were determined by absorbance at 260 nm (OD260). The calculation formula thereof was: virus concentration = OD260 × dilution multiple × 36/genome length (Kb). The virus stock solution was cryopreserved at -80°C.

### Detection of Spike gene expression

According to a conventional method, transfecting 2.5 µg of pGA-NB1, pGA-NB2 and pGA-NB4, respectively, with cationic liposomes for 48 hours, and then collecting the cells. Infecting HEK293 cells with Ad5-NB2 and the Ad5 empty vector viruses, and, after 24 hours, collecting the cells. Such four samples were processed according to a conventional Western Blot method, and were detected for the protein (FIG. 1).

It could be seen that no S protein expression was detected in the pGA1-NB1 sample, while the expression of the S protein could be observed in the codon-optimized pGA1-NB2, pGA1-NB4 and vaccine candidate strain Ad5-NB2 samples. This indicates that the NB2 sequence produces unexpected effect. The expression of the S protein could also be observed in pGA1-NB4. It indicates that a nucleotide sequence having at least 85%, further 86%, 90%, 95%, 98% and more sequence identity with NB2 has a limited expression of the corresponding protein.

### Animal immunogenicity evaluation

Balb/c mice, 6-8 weeks old, were divided into 5 groups with 5 mice in each group. On day 0, the mice were intramuscularly immunized with Ad5-NB2 at 5×10⁹ vp/animal, through intramuscular or intra-nasal administration. On day 8, blood was taken from orbit, and serum was separated. The antibody level in the serum was detected by adopting the S protein of the novel coronavirus as the antigen through enzyme-linked immunosorbent assay (ELISA). The specific process were as follows:
1) adding 100 µl of PBS and 50 ng of the S protein per well in a 96-well plate, incubating overnight at 4°C, for 16-18 h;
2) removing the supernatant, washing with PBST once, adding 200 µl of 5% skim milk for blocking at room temperature for 2 h;
3) washing with PBST twice;
4) adding the samples: adding 100 µl of diluted serum samples, incubating at 37°C for 2 h, and then washing with PBST for 5 times;
5) adding enzyme-labeled antibodies: adding 100 µl of diluted HRP-labeled IgM or IgG secondary antibody, and incubating at 37°C for 2 h;
6) washing with PBST for 6-8 times;
7) adding substrate solution for development: adding 100 µl of TMB for development;
8) terminating the reaction: adding with 50 µl of 1 M sulfuric acid to terminate the reaction; and
9) determining the results: determining the OD values, and controlling the OD values between 0.1 and 4.

The experimental results showed that, Ad5-NB2 could induce the generation of antibodies in the mice (FIG. 2).

### Immunoprotection evaluation

The macaque monkeys were obtained from Guangdong Landau Biotechnology Co. Ltd. The vaccinated macaque monkeys were 6 to 14 years old.

The macaque monkeys were randomly divided into 4 groups, with 4 monkeys in each group, as shown in the following table:

| Groups No. | *Macaca mulatta* | Gender | Immunization dose/mode | Time for virus challenge (days after immunization) |
|---|---|---|---|---|
| 1 | 063585 | Male | Ad5-NB2 (1 × 10¹⁰ vp) Intramuscular (IM) injection | 30 |
| | 071515 | Male | | |
| | 080066 | Female | | |
| | 134056 | Female | | |
| 2 | 100109 | Male | Ad5-NB2 (1 × 10¹¹ vp) Intramuscular (IM) injection | 56 |
| | 116004 | Male | | |
| | 116008 | Female | | |
| | 130460 | Female | | |
| 3 | 071539 | Male | Ad5-NB2 (1 × 10¹¹ vp), intra-nasal/oral (I.N.) | 30 |
| | 110113 | Male | | |
| | 140052 | Female | | |
| | 134018 | Female | | |
| 4 | 100886 | Female | Ad5-empty (1 × 10¹¹ vp) | 30 |
| | 110075 | Male | | |
| 5 | C1 | Female | **-** | - |
| | C2 | Male | | |
| | C3 | Female | | |
| | C4 | Male | | |
| | D1 | Female | | |
| | D2 | Male | | |

| | | | | |
|---|---|---|---|---|
| Note: the intra-nasal/oral immunizing dose for each macaque monkey was 5 × 10¹⁰ vp. | | | | |

Macaque monkeys were immunized with the vaccine prepared from the Ad5-NB2 virus strain. Blood was taken at days 12, 18 and 24 after the immunization, to determine the antibody binding titers by ELISA, and the neutralizing antibody titers by the SARS-CoV-2 virus. The peripheral blood was isolated and detected for cellular immune reaction by ELISpot assay.

### Experimental results

### (1) Binding antibodies

12 days after the intramuscular (I.M.) vaccination, significant presence of S-specific IgG was detected in the sera of 4 macaque monkeys injected with 1 × 10¹¹ vp and 3 macaque monkeys injected with 1×10¹⁰ vp. After 24 days, S-specific IgG was detected in the sera of all immunized macaque monkeys. In the macaque monkeys immunized with a low or high dose vaccine, the S-specific IgG increased continuously over time (FIG. 3a).
12 days after the intra-nasal/oral vaccination, the S-specific IgG was only detected in the sera of 3 out of 4 vaccinated macaque monkeys. On 24th day, the S-specific IgG was detected in the sera of all immunized macaque monkeys.

Compared with intramuscular vaccination, the serum IgG titer reached the peak on 18th day after intra-nasal/oral vaccination, 1-2 logarithms lower than that of the macaque monkeys intramuscularly immunized with the same dose of the vaccine (FIGs. 3a and 3b).

### (2) Neutralizing Antibodies

The neutralizing antibody titers of the serum samples were determined by means of the neutralization reaction between a live novel coronavirus (2019-nCoV-WIV04 strain) and serum antibodies.

Heat-inactivating the obtained animal serum samples at 56°C for 30 min; diluting by 1:50, 1:150, 1:450, 1:1350, 1:4050 and 1:12150, respectively; adding the equal amount of live viruses and incubating at 37°C under 5% CO₂ for 3 days; and then fixing the cells with 4% formaldehyde and staining with crystal violet. The number of plaques was counted to determine the neutralizing antibody titer (EC₅₀).

The results showed (FIG. 4) that: 28 days after the immunization, the sera of the macaque monkeys injected intramuscularly with 1 × 10¹¹ vp had neutralizing antibodies against live novel coronaviruses; and the sera of the macaque monkeys with intra-nasal/oral immunization (5 × 10¹⁰ vp/animal) also had neutralizing antibodies against live novel coronaviruses, which had lower titer than that of the intramuscularly immunized macaque monkeys. 56 days after the immunization, the sera of the macaque monkeys injected intramuscularly with 1 × 10¹⁰ vp also had neutralizing antibodies against live novel coronaviruses, which had lower titer than that of the macaque monkeys injected intramuscularly with 1 × 10¹⁰ vp.

### (3) Cellular Immunity

To determine whether Ad5-NB2 could also induce a cellular immune response in non-human primates (NHPs), we detected the response of S-specific IFN-γ secreting cells from the peripheral blood mononuclear cells (PBMCs), to S1 and S2 peptide libraries.

The results showed that:
1) On the 18th day after the intramuscular vaccination, all 8 macaque monkeys had cellular immune responses to the S1 peptide library, and 6 macaque monkeys had cellular immune responses to the S2 peptide library (FIG. 5).
2) On the 18th day after the intra-nasal/oral vaccination, 2 out of 4 vaccinated macaque monkeys showed weak cellular immune responses to the S1 peptide library, and all 4 vaccinated macaque monkeys had no obvious response to the S2 peptide library on the 18th day (FIG. 5).

Therefore, in the macaque monkeys, the cellular immune response was mainly directed at the S1 region, which was similar to that observed in mice. These results indicates that the intramuscular immunization of the vaccine can cause systemic cellular immune response to the S protein, especially to the S1 region. While the mucosal vaccination of the vaccine causes weaker systemic cellular immune response.

### Protective efficacy test (virus challenge tests in macaque monkeys)

The virus challenge tests were carried out in the P4 Laboratory of Wuhan Institute of Virology, Chinese Academy of Sciences. At 30 days for the group immunized with 1 × 10¹¹ VP (IM. and I.N. vaccination groups), and 8 weeks for the group immunized with a low dose of 1 × 10¹⁰ VP (IM. vaccination group) after the vaccination, the animals were challenged with 2 × 10⁴ TCID50 SARS-CoV-2 (2019-nCoV-WIV04) by intratracheal route. Due to the limited space of the P4 Laboratory, we only randomly selected 3 macaque monkeys of the I.M. high-dose vaccinated group, 3 macaque monkeys of the I.N. high-dose vaccinated group, and 3 macaque monkeys of the I.M. low-dose vaccinated group, for the virus challenge test. At the same time, 4 non-vaccinated macaque monkeys (C1-C4) received the virus challenge with the same dose of viruses, and the other 2 non-vaccinated macaque monkeys (D1 and D2) received the virus challenge at the dose of 400 TCID50. The nucleic acids from ten sites, such as pharyngeal swab, bronchus and lung lobe, were collected and detected for 10 consecutive days after the virus challenge. The detection results showed that:
1) With genome detection of the novel coronaviruses, no virus was detected at the pharyngeal swabs of the immunized macaque monkeys after virus challenge, or a very low number of copies of the virus was detected at some sites.
   - For the non-vaccinated macaque monkeys (C1-C4, D1-D2), a high load of novel coronavirus nucleic acids was detected in the pharyngeal swabs, and the viral load in the pharyngeal swabs climbed to a peak of 4.2 × 10⁶ copies/ml on the 5th day after the virus challenge (FIG. 6 a).
   - For the intramuscular vaccination group of a high dose of 1 × 10¹¹ vp, the nucleic acids on the pharyngeal swabs were detected by qPCR quantitative tests 30 days after immunization. The results showed that, the viral loads of some of the pharyngeal swabs of the macaque monkeys were slightly higher than the detection limit only on day 1 or day 5, and the other viral loads were undetectable. Therefore, the SARS-CoV-2 infection can be effectively prevented by intramuscular vaccination of 1 × 10¹¹ vp of Ad5-NB2 (FIG. 6b).
   - For the intramuscular vaccination group of a low dose of 1 × 10¹⁰ vp, the nucleic acids on the pharyngeal swabs were detected by qPCR quantitative tests 56 days (8 weeks) after immunization. The result showed that the viral loads of some of the pharyngeal swabs of the macaque monkeys were slightly higher than the detection limit only on day 3 or day 5, and the other viral loads were undetectable. Therefore, the SARS-CoV-2 infection can be effectively prevented by intramuscular vaccination of 1 × 10¹⁰ vp of Ad5-NB2 (FIG. 6c).
   - For the intra-nasal/orally immunized macaque monkeys, the virus was undetectable in all of macaque monkeys in the first 4 days after the virus challenge, and the viral loads from some of the macaque monkeys were slightly higher than the detection limit on day 6 or day 7 and then decreased, and were undetectable on day 10. Therefore, the intra-nasal/oral immunization can also effectively resistant to the SARS-CoV-2 infection (FIG. 6d).
      In view of the above, all immunized macaque monkeys were fully protected, and effectively resistant to the SARS-CoV-2 infection. The systemic antibodies and cellular immune responses induced by the intramuscular vaccination in the macaque monkeys was significantly higher than those induced by the mucosal vaccination.
2) No sign was found for the antibody-dependent enhancement (ADE) in all vaccinated macaque monkeys after virus challenge.
   We calculated the viral loads based on the area under curve (AUC). The AUCs (2.7 ± 0.6 log₁₀) of the viruses of the vaccinated macaque monkeys was 2500 times lower than those (6.1 ± 1.0 logic) of the non-vaccinated macaque monkeys (see FIG. 7).
3) After euthanasia, the virus genomes in the biopsy samples from nine anatomic sites (including trachea, left and right bronchi, upper, middle and lower parts of left and right lungs) of the macaque monkeys were evaluated.
   The results showed that: the virus genomes were detected in the left bronchus (2.4 × 10⁵ copies/ml) and the lower part of left lung (1.8 × 10⁴ copies/ml) of the non-vaccinated macaque monkey C3; the virus genomes were also detected in the trachea (4.7 × 10⁴ copies/ml), left (2.2 × 10⁴ copies/ml) and right bronchi (1.8 × 10⁴ copies/ml) of the non-vaccinated macaque monkey C4. In contrast, it showed that the results were all negative through the novel coronavirus nucleic acid test performing on 81 biopsy samples collected from 9 vaccinated macaque monkeys (FIG. 8).
4) Pathological sections confirmed that the lungs of the immunized macaque monkeys were effectively protected.
   Histopathological analysis was carried out on the lung slices of the non-vaccinated and vaccinated macaque monkeys. In the non-SARS-CoV-2-vaccinated macaque monkeys, SARS-CoV-2 intratracheal inoculation caused severe interstitial pneumonia, which was manifested by alveolar septum dilatation, infiltration of monocytes and lymphocytes in most alveoli, and a certain proportion of alveolar edema. In the vaccinated macaque monkeys, no obvious pathological abnormality was caused by the virus challenge test. Some of the macaque monkeys had slight histopathological changes in the lungs, which might be due to mechanical damages caused by direct endotracheal intubation for performing virus challenge to the macaque monkeys (FIG. 9).
5) Titer changes of serum antibody of the macaque monkeys before and after immunization
   A plaque reduction neutralization test (PRNT) was performed for comparing the titers of neutralizing antibodies in the sera before and after the virus challenge.
   - All non-vaccinated macaque monkeys did not show SARS-CoV-2-neutralizing activity in sera before virus challenge or at 7 days after virus challenge (< 1:50) (FIG. 10d).
   - For the intramuscular vaccination group of a high dose, the titers of neutralizing antibodies in the sera were respectively 1:636, 1:389 and 1:784 before virus challenge, and were respectively 1:1225, 1:518 and 1:1350 on day 7 after virus challenge. It indicates that the virus challenge has no or low enhancement effect (FIG. 10a).
   - For the intramuscular vaccination group of a low dose, only one macaque monkey (No. 080066) had significantly increased titer of neutralizing antibody in the serum (IC50 1:188 → 1:1460) on day 7 after virus challenge, while the other two macaque monkeys had no increase in the titer of neutralizing antibodies in the sera (IC50 1:315 → 1:192, 1: 400 → 1:330), as compared with those before the virus challenge (FIG. 10c).
   - For the intra-nasal/oral vaccination group, the titers of neutralizing antibodies in the sera were relatively low both before the virus challenge and on day 7 after the virus challenge, without significant change. The titers of neutralizing antibodies in the sera were respectively 1:164, 1:137 and 1:150 before virus challenge, and were respectively 1:193, 1:168 and 1:130 on day 7 after virus challenge (FIG. 10b).
   - The levels of anti-Ad5 neutralizing antibodies were detected in the Ad5-NB2-immunized macaque monkeys. The results showed that the titers of Ad5-neutralizing antibodies in the sera were increased on day 6 after Ad5-NB2 immunization in the intramuscular vaccination group. However, for the intra-nasal/orally Ad5-NB2-immunized group, the titers of Ad5-neutralizing antibodies in the sera were not increased significantly on day 6 after the immunization, and were increased on day 24, but not higher than those in the group of a low dose (FIG. 11a-11d).

In view of the above, in 6 out of 9 macaque monkeys from the immunized groups, the titers of neutralizing antibodies against the novel coronavirus did not increase significantly before and after the virus challenge. It suggested that the virus was eliminated immediately and no obvious virus replication occured.

In summary, the optimized S gene, NB2, in the replication-defective type 5 adenovirus vector can significantly improve antigen expression, and induce high-titer antibodies and cellular immunity after immunizing mice and macaque monkeys *(Macaca mulatta).* The macaque monkeys were immunized by single intramuscular or intra-nasal/oral administration of the vaccine, and were fully resistant to the virus infection after being subjected to virus challenge 30 days after the immunization. When the macaque monkeys were intramuscularly immunized once with 1/10 low dose (1 × 10¹⁰ vp/animal) of the vaccine and then subjected to virus challenge 8 weeks after the immunization, the macaque monkeys were also fully resistant to the virus infection. When the vaccinated macaque monkeys were subjected to virus challenge, we did not observe lung injury caused by ADE or virus. In most of the vaccinated macaque monkeys, the antibody level did not increase after virus challenge, which indicated that the virus was eliminated quickly and did not cause stimulation effect on the immune system. Different from the whole-virus inactivated vaccine, the vaccine of the present disclosure, in addition to stimulating the humoral immunity of a body, also induces cellular immunity, which can further improve the protective effect on body function. Furthermore, since the nasal and oral immunization also obtained good protective effect, it suggests the feasibility of mucosal- and non-injection immunization.

## Claims

1. A vaccine for preventing Novel Severe Acute Respiratory Syndrome Coronavirus 2 (SARS-CoV-2) infection, comprising:
a nucleotide sequence as shown by SEQ ID NO: 1; or
a nucleotide sequence having at least 80%, 85%, 90%, 95% or 100% homology with the nucleotide sequence as shown by SEQ ID NO: 1.

2. The vaccine according to claim 1, wherein the nucleotide sequence is capable of expressing a protein in a human-derived cell or human body.

3. The vaccine according to claim 2, wherein, in the human body, the protein is capable of:
inducing an immune response;
generating a molecular biology reporter;
generating a trace molecule for detection;
regulating a gene function; or
functioning as a therapeutic molecule.

4. The vaccine according to any one of claims 1 to 3, further comprising a pharmaceutically-acceptable adjuvant, carrier, diluent or excipient.

5. The vaccine according to any one of claims 1 to 3, further comprising at least one medicament having a therapeutic effect on coronavirus disease 2019 (COVID-19).

6. An expression vector comprising the nucleotide sequence according to claim 1.

7. The expression vector according to claim 6, wherein the vector is a DNA plasmid, an RNA expression plasmid or a viral vector.

8. The expression vector according to claim 7, wherein the viral vector is an adenovirus vector.

9. The adenovirus vector according to claim 8, wherein the nucleotide sequence according to claim 1 has a transcription direction opposite to those of other genes in the vector.

10. Use of the vaccine according to claim 1, comprising:
preparing a COVID-19 detection reagent; and
preparing a gene-function regulator.

11. A method for preventing or treating COVID-19, comprising administering a prophylactically or therapeutically effective amount of the vaccine according to any one of claims 1-5 to a human.
